(19) Europäisches Patentamt
European Patent Office
Office européen des brevets

(11) EP 0 871 426 B1

(12) **EUROPÄISCHE PATENTSCHRIFT**

(45) Veröffentlichungstag und Bekanntmachung des Hinweises auf die Patenterteilung:
**09.07.2003 Patentblatt 2003/28**

(21) Anmeldenummer: **97913130.7**

(22) Anmeldetag: **29.09.1997**

(51) Int Cl.[7]: **A61K 7/13**

(86) Internationale Anmeldenummer:
**PCT/EP97/05341**

(87) Internationale Veröffentlichungsnummer:
**WO 98/017234 (30.04.1998 Gazette 1998/17)**

(54) **MITTEL UND VERFAHREN ZUR FÄRBUNG KERATINISCHER FASERN**

AGENT AND METHOD FOR DYEING KERATIN FIBRES

AGENT ET PROCEDE POUR LA COLORATION DE FIBRES KERATINIQUES

(84) Benannte Vertragsstaaten:
**DE ES FR GB IT**

(30) Priorität: **18.10.1996 DE 19643059**

(43) Veröffentlichungstag der Anmeldung:
**21.10.1998 Patentblatt 1998/43**

(73) Patentinhaber: **Wella Aktiengesellschaft**
**64274 Darmstadt (DE)**

(72) Erfinder: **BRAUN, Hans-Jürgen**
**CH-3182 Überstorf (CH)**

(56) Entgegenhaltungen:
**EP-A- 0 634 162**
**EP-A- 0 667 143**
**EP-A- 0 687 669**
**DE-A- 3 610 396**
**DE-A- 4 400 757**
**DE-A- 4 422 603**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft eine Zusammensetzung zur Färbung von keratinischen Fasern, insbesonders von menschlichen Haaren. Diese Zusammensetzungen enthalten wenigstens ein 4,5-Diaminopyrazol, ein m-Aminophenol und ein m-Phenylendiamin. Die Färbungen der keratinischen Fasern werden ausgeführt, indem die oben erwähnte Zusammensetzung mit einem Oxidationsmittel vermischt, auf die keratinischen Fasern aufgetragen wird und nach einer bestimmten Einwirkungszeit mit Wasser und einem Shampoo wieder ausgewaschen wird.

Für eine bestimmte Gruppe von Anwendungen besteht eine Nachfrage nach Zusammensetzungen, die Haare in kupferfarbigen, kastanienbraunen bis roten Nuancen zu färben vermögen. Diese Nachfrage wird heute befriedigt, indem auf dem Markt Haarfärbemittel mit einem Gehalt an bestimmten Oxidationsfarbstoffvorstufen angeboten werden. Beispielsweise wird in der DE-OS 36 10 396 zur Erzeugung von neutralen roten Farbtönen die Verwendung einer Kombination aus 5-Amino-2-methylphenol, 4-Amino-3-methylphenol und 1,4-Diaminobenzol und/oder 2,5-Diaminotoluol vorgeschlagen. In der EP-OS 0 634 162 werden Beispiele zur Kombination von 5-Amino-2-methylphenol und dessen N-substituierten Derivaten, 4-Amino-2-methylphenol, 4-Amino-3-methylphenol oder 4-Amino-2-hydroxymethylphenol und bestimmten m-Phenylendiaminderivaten beschrieben.

Die Beständigkeit der nach dem Stand der Technik erhaltenen Haarfärbungen gegenüber Lichteinwirkung, Auswaschen, Wettereinflüssen. Schweiß und anderen Haarbehandlungen ist jedoch ungenügend.

Es wurde nunmehr überraschend gefunden, daß die geschilderten Nachteile vermieden werden, wenn für eine oxidative Haarfärbung im rötlichen Farbbereich eine Kombination der folgenden Farbvorstufen verwendet wird:

- mindestens ein 4,5-Diaminopyrazol gemäß allgemeiner Formel (I),

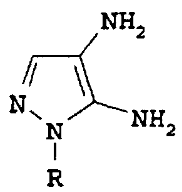

I

,

worin R eine geradkettige oder verzweigte Alkylgruppe mit 1 bis 6 Kohlenstoffatomen, eine geradkettige oder verzweigte Mono- oder Polyhydroxyalkylgruppe mit 2 bis 6 Kohlenstoffatomen oder eine geradkettige oder verzweigte Mono- oder Polyaminoalkylgruppe, oder dessen Salze mit kosmetisch verträglichen Säuren;

- mindestens ein 5-Amino-2-methylphenolderivat gemäß allgemeiner Formel (II),

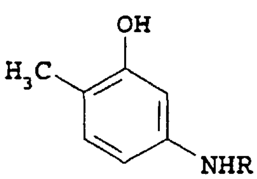

II

,

worin R' gleich Wasserstoff, einer geradkettigen oder verzweigten Alkylgruppe mit 1 bis 4 Kohlenstoffatomen, einer geradkettigen oder verzweigten Hydroxyalkylgruppe mit 2 bis 4 Kohlenstoffatomen oder einer geradkettigen oder verzweigten Polyhydroxyalkylgruppe mit 3 bis 4 Kohlenstoffatomen ist;

- mindestens ein m-Phenylendiaminderivat gemäß allgemeiner Formel (III),

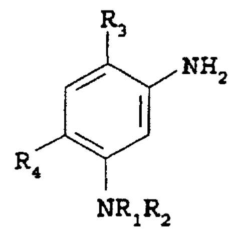

III

,

worin $R_1$ und $R_2$ unabhängig voneinander ein Wasserstoffatom, eine geradkettige oder verzweigte Alkylgruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeuten,

$R_3$ ein Wasserstoffatom, eine geradkettige oder verzweigte Alkylgruppe mit 1 bis 6 Kohlenstoffatomen, eine geradkettige oder verzweigte Alkoxygruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet, und

$R_4$ unabhängig von $R_1$, $R_2$ und $R_3$ ein Wasserstoffatom, eine geradkettige oder verzweigte Alkylgruppe mit 1 bis 6 Kohlenstoffatomen, eine geradkettige oder verzweigte Alkoxygruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet.

Die Synthese und die Verwendung von Verbindungen gemäß allgemeiner Formel (I) in Oxidationshaarfärbemitteln ist im Prinzip bekannt aus der DE-OS 42 34 885. Die in der vorliegenden Anmeldung beschriebenen neuen Kombinationen mit Haarfarbvorstufen weisen jedoch überraschende Vorteile bezüglich der Stabilität der erhaltenen Färbungen auf.

Bevorzugte Derivate gemäß allgemeiner Formel (I) sind die Verbindungen 4,5-Diamino-1-methyl-1H-pyrazol, 4,5-Diamino-1-ethyl-1H-pyrazol, 4,5-Diamino-1-(1'-methylethyl)-1H-pyrazol und 4,5-Diamino-1-(2'hydroxyethyl)-1H-pyrazol.

Bevorzugte Derivate gemäß allgemeiner Formel (II) sind die Verbindungen 5-Amino-2-methylphenol, 5-Methylamino-2-methylphenol und 5-[(2'-Hydroxyethyl)amino]-2-methylphenol.

Bevorzugte Derivate gemäß allgemeiner Formel (III) sind die Verbindungen 2,4-Diamino-1-(2'-hydroxyethoxy) benzol, 2,4-Diamino-1-(2',3'dihydroxypropoxy)benzol, 1,3-Bis(2',4'-diaminophenoxy)propan, 2-Amino-4-[(2'-hydroxyethyl)-amino]anisol, 1,5-Bis(2'-hydroxyethoxy)-2,4-diaminobenzol und m-Phenylendiamin.

Zur Abrundung des Farbergebnisses sowie zur Erzeugung von speziellen Farbeffekten können der vorgenannten Farbstoffkombination weitere Oxidationsfarbvorstufen wie Derivate des p-Phenylendiamins wie 2-(2',5'-Diaminophenyl)ethanol, Resorcinderivate wie Resorcin oder 4-Chlorresorcin, Amino- und Hydroxyderivate von 1,3-Benzodioxol, Naphthalinderivate wie 1-Hydroxynaphthalin, 1,5- Dihydroxynaphthalin oder 1,7-Dihydroxynaphthalin sowie direktziehende Farbstoffe, wie zum Beispiel 2-Amino-4,6-dinitrophenol, 2-Ethylamino-4,6-dinitrophenol, 2-[(2'-Hydroxyethyl)amino]-4,6-dinitrophenol, 2-Amino-6-chlor-4-nitrophenol oder 2-Chlor-6-ethylamino-4-nitrophenol zugesetzt werden.

Die vorstehend beschriebenen erfindungsgemäßen Kombinationen von oxidativen Haarfarbvorstufen und gegebenenfalls direktziehenden Farbstoffen werden zur Färbung in einer geeigneten Farbträgermasse appliziert.

Der Gegenstand der vorliegenden Anmeldung betrifft daher auch ein Mittel zur oxidativen Färbung von Haaren, welches durch Vermischen einer Farbträgermasse mit einem Oxidationsmittel unmittelbar vor der Anwendung hergestellt wird.

Die erfindungsgemäße Farbträgermasse enthält die vorstehend beschriebenen erfindungsgemäßen Kombinationen von oxidativen Haarfarbstoffen und gegebenenfalls direktziehenden Farbstoffen als solche oder in Form von physiologisch verträglichen Salzen, beispielsweise Hydrochloride oder Sulfate oder im Fall von Phenolen als Alkaliphenolate.

Die Gesamtkonzentration an Farbvorstufen beträgt 0,1 bis 10 Gewichtsprozent, bevorzugt 0,2 bis 6 Gewichtsprozent. Die Konzentration der einzelnen Haarfarbstoffe beträgt 0,01 bis 5 Gewichtsprozent, vorzugsweise 0,1 bis 4 Gewichtsprozent.

Darüberhinaus können in der Farbträgermasse noch übliche kosmetische Zusätze, beispielsweise Antioxidantien wie Ascorbinsäure, Thioglykolsäure oder Natriumsulfit; Parfümöle; Komplexbildner; Netzmittel; Emulgatoren; Verdicker; Pflegestoffe und andere vorhanden sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt

eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Das gebrauchsfertige erfindungsgemäße Haarfärbemittel wird durch Mischen der Farbträgermasse mit einem flüssigen Oxidationsmittel unmittelbar vor der Anwendung hergestellt.

Die Farbträgermasse und das Oxidationsmittel werden hierbei im Gewichtsverhältnis von 5:1 bis 1:3 miteinander vermischt, wobei ein Gewichtsverhältnis von 1:1 bis 1:2 besonders bevorzugt ist.

Der pH-Wert des gebrauchsfertigen erfindungsgemäßen Haarfärbemittel stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis beinflußt wird. Der pH-Wert des fertigen Haarfärbemittels liegt bei 3 bis 11, vorzugsweise 5 bis 9.

Für die Einstellung des jeweiligen pH-Wertes der Farbträgermasse und des Oxidationsmittels können je nach dem gewünschten pH-Wert organische und anorganische Säuren, wie zum Beispiel Phosphorsäure, Ascorbinsäure und Milchsäure, oder Alkalien wie zum Beispiel Monoethanolamin, Triethanolamin, 2-Amino-2-methyl-1-propanol, Ammoniak, Natronlauge, Kalilauge oder Tris-(hydroxymethyl)-amino-methan, verwendet werden.

Für die Anwendung zur oxidativen Färbung von Haaren vermischt man die vorstehend beschriebene Farbträgermasse unmittelbar vor dem Gebrauch mit einem Oxidationsmittel und trägt eine für die Haarfärbebehandlung ausreichende Menge, je nach Haarfülle im allgemeinen etwa 60 bis 200 Gramm des erhaltenen gebrauchsfertigen Oxidationshaarfärbemittels auf das Haar auf.

Als Oxidationsmittel kommen hauptsächlich Wasserstoffperoxid oder dessen Additionsverbindungen an Harnstoff, Melamin oder Natriumbromat in Form einer 1 bis 12prozentigen, vorzugsweise 6prozentigen, wäßrigen Lösung in Betracht, wobei Wasserstoffperoxid besonders bevorzugt wird.

Man lässt das erfindungsgemäße Haarfärbemittel bei 15 bis 50 Grad Celsius etwa 10 bis 45 Minuten lang, vorzugsweise 30 Minuten lang, auf das Haar einwirken, spült sodann das Haar mit Wasser und trocknet es. Gegebenenfalls wird im Anschluss an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die nachfolgenden Beispiele sollen den Gegenstand näher erläutern, ohne ihn auf diese Beispiele zu beschränken:

## Beispiele

**Beispiele 1 bis 6:** Haarfärbelösungen mit alkalischem pH-Wert

Es wird folgende Farbelösung hergestellt:

| | |
|---|---|
| 10,0 g | Isopropanol |
| 10,0 g | Laurylalkohol-diglykolether-sulfat-Natriumsalz, (28-prozentige wäßrige Lösung) |
| 10,0 g | Ammoniak 25-prozentige wäßrige Lösung |
| 0,3 g | Ascorbinsäure |
| X g | Farbvorstufen gemäß Tabelle 1 |
| ad 100 g | Wasser vollentsalzt |

Zur Anwendung werden 10 g Haarfärbelösung mit 10 g Wasserstoffperoxid-Lösung (6prozentige Lösung in Wasser) gemischt. Das erhaltene Oxidationshaarfärbemittel wird auf Haarsträhnchen aufgetragen. Nach einer Einwirkungszeit von 30 Minuten bei 40 °C werden die Haare mit Wasser gespült, schamponiert und getrocknet.

Tabelle 1

| **Farbvorstufen/Beispiel** | **1** | **2** | **3** | **4** | **5** | **6** |
|---|---|---|---|---|---|---|
| 4,5-Diamino-1-(1'-methylethyl)-1H-pyrazol-sulfat | 1,5 g- | - | 2,1 g | - | 1,9 g | - |
| 4,5-Diamino-1-(2'-hydroxyethyl)-1H-pyrazolsulfat | - | 2,0 g | - | 2,6 g | - | 3,5 g |
| 5-Amino-2-methyl-phenol | 1,0 g | 1,2 g | 0,6 g | 0,6 g | - | 0,3 g |
| 5-[(2'-Hydroxyethyl)amino]-2-methyl-phenol | - | - | - | - | 0,8 g | 0,5 g |
| 2,4-Diamino-1-(2'-hydroxyethoxy)-benzoldihydrochlorid | - | 0,3 g | 1,2 g | - | 1,2 g | 0,1 g |
| 1,3-Bis(2',4'-diaminophenoxy)-propantetrahydrochlorid | 0,3 g- | - | - | - | - | 0,5 g |
| 2-Amino-4-[(2'-hydroxyethyl) amino]-anisol-sulfat | 0,3 g | 0.4 g | - | - | - | 1,2 g |
| 1,5-Bis(2'-hydroxyethoxy)-2,4-diamino-benzoldihydrochlorid | | - | - | 1,5 g | - | - |
| **Farbe auf gebleichtem Tierhaar** | bordeaux | weinrot | Cyclamen | Cyclamen | Cyclamen | modischrotbraun |

**Beispiel 7:** Haarfärbemittel in Cremeform

| | |
|---|---|
| 0,52 g | 2,5-Diaminophenylethylalkohol-sulfat |
| 0,95 g | 4,5-Diamino-1-(2-hydroxyethyl)-1H-pyrazol-sulfat |
| 0,24 g | Resorcin |
| 0,30 g | m-Aminophenol |
| 0,24 g | 2-Amino-4-(2'-hydroxyethyl)aminoanisolsulfat |
| 0,20 g | 5-Amino-2-methyl-phenol |
| 0,05 g | 2-Amino-6-chlor-4-nitrophenol |
| 0,12 g | 1-Naphthol |
| 15,00 g | Cetylalkohol |
| 3,50 g | Natrium-Laurylalkohol-diglykolethersulfat, 28prozentige wäßrige Lösung |
| 3,00 g | Ammoniak, 25prozentige wäßrige Lösung |
| 0,30 g | Natriumsulfit, wasserfrei |
| 75,58 g | Wasser |
| 100,00 g | |

10 g dieses Haarfärbemittels werden kurz vor Gebrauch mit 10 ml Wasserstoffperoxidlösung (6prozentig) vermischt. Anschließend trägt man das Gemisch auf blonde Naturhaare auf und läßt es 30 Minuten lang bei 40 Grad Celsius einwirken. Danach wird das Haar mit Wasser gespült und getrocknet. Das Haar hat eine rotbraune Färbung erhalten.

**Beispiel 8:** Haarfärbemittel in Gelform

| | |
|---|---|
| 1,5 g | 5-Amino-2-methyl-phenol |
| 1,0 g | 4,5-Diamino-1-(2'-hydroxyethyl)-1H-pyrazol-sulfat |
| 0,6 g | 2,5-Diamino-toluol-sulfat |
| 0,5 g | 2-Chlor-6-ethylamino-4-nitrophenol |
| 0,4 g | Natriumhydroxid, fest |
| 0,6 g | Ascorbinsäure |
| 7,0 g | Isopropanol |
| 15,0 g | Ölsäure |
| 10,0 g | Ammoniak, 25prozentige wäßrige Lösung |
| 62,5 g | Wasser |
| 100,0 g | |

Man vermischt kurz vor dem Gebrauch 50 g dieses Haarfärbemittels mit 50 ml Wasserstoffperoxidlösung (6prozentig) und läßt das Gemisch 30 Minuten lang auf blonde menschliche Haare einwirken. Danach wird mit Wasser gespült und sodann getrocknet. Man erhält eine rotbraune Haarfärbung.

**Beispiel 9:** Haarfärbelösung mit saurem pH-Wert

| | |
|---|---|
| 0,30 g | 2-(2',5'-Diaminophenyl)ethanol-sulfat |
| 0,18 g | 4-Chlorresorcin |
| 0,30 g | 4,5-Diamino-1-(2'-hydroxyethyl)-1H-pyrazol-sulfat |
| 0,30 g | 1-Naphthol |
| 0,30 g | Ascorbinsäure |
| 0,28 g | Natriumlaurylethersulfat |
| 0,22 g | Ammoniak, 25prozentige wäßrige Lösung |
| 98,12 g | Wasser |
| 100,00 g | |

Der pH-Wert der Farbträgermasse wird mit verdünnter Phosphorsäure oder einer verdünnten Ammoniaklösung auf einen pH-Wert von 6,8 eingestellt.

Unmittelbar vor der Anwendung werden 20 Gramm der Haarfärbelösung mit 20 Gramm einer 6prozentigen, wäßrigen Wasserstoffperoxidlösung (pH = 6,8) vermischt und das erhaltene Oxidationshaarfärbemittel (pH = 6,8) auf gebleichte Haare aufgetragen. Nach einer Einwirkungszeit von 30 Minuten bei Raumtemperatur werden die Haare mit Wasser gespült und getrocknet.

Das so behandelte Haar ist in einem rotvioletten Farbton gefärbt.

**Patentansprüche**

1. Haarfarbträgermasse enthaltend

- mindestens ein 4,5-Diaminopyrazol gemäß allgemeiner Formel (I),

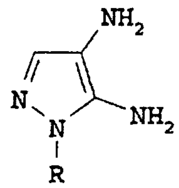 I

,

worin R eine geradkettige oder verzweigte Alkylgruppe mit 1 bis 6 Kohlenstoffatomen, eine geradkettige oder verzweigte Mono- oder Polyhydroxyalkylgruppe mit 2 bis 6 Kohlenstoff-atomen oder eine geradkettige oder verzweigte Mono- oder Polyaminoalkylgruppe, oder dessen Salze mit kosmetisch verträglichen Säuren darstellt;

- mindestens ein 5-Amino-2-methylphenolderivat gemäß allgemeiner Formel (II),

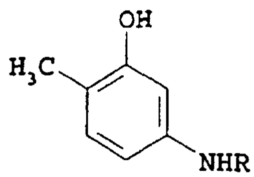 II

,

worin der Rest R' die Bedeutung von Wasserstoff, einer geradkettigen oder verzweigten Alkylgruppe mit 1 bis 4 Kohlenstoffatomen, einer geradkettigen oder verzweigten Hydroxyalkylgruppe mit 2 bis 4 Kohlenstoffatomen oder einer geradkettigen oder verzweigten Polyhydroxyalkylgruppe mit 3 bis 4 Kohlenstoffatomen hat;

- mindestens ein m-Phenylendiaminderivat gemäß allgemeiner Formel (III),

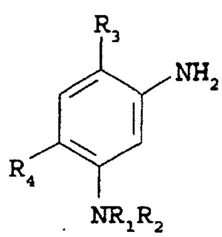 III

,

worin $R_1$ und $R_2$ unabhängig voneinander ein Wasserstoffatom, eine geradkettige oder verzweigte Alkylgruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeuten, $R_3$ ein Wasserstoffatom, eine geradkettige oder verzweigte Alkylgruppe mit 1 bis 6 Kohlenstoffatomen, eine geradkettige oder verzweigte Alkoxygruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet, und $R_4$ unabhängig von $R_1$, $R_2$ und $R_3$ ein Wasserstoffatom, eine geradkettige oder verzweigte Alkylgruppe mit 1 bis 6 Kohlenstoffatomen, eine geradkettige oder verzweigte Alkoxygruppe mit 1 bis 4 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet.

**2.** Haarfarbträgermasse gemäß Anspruch 1, **dadurch gekennzeichnet, daß** das 4,5-Diaminopyrazolderivat ausgewählt ist aus 4,5-Diamino-1-methyl-1H-pyrazol, 4,5-Diamino-1-ethyl-1H-pyrazol, 4,5-Diamino-1-(1'-methylethyl)-1H-pyrazol und 4,5-Diamino-1-(2'-hydroxyethyl)-1H-pyrazol.

3. Haarfarbträgermasse gemäß Anspruch 1, **dadurch gekennzeichnet, daß** das 5-Amino-2-methylphenolderivat ausgewählt ist aus 5-Amino-2-methylphenol, 5-Methylamino-2-methylphenol und 5-[(2'-Hydroxyethyl)amino]-2-methylphenol.

4. Haarfarbträgermasse gemäß Anspruch 1, **dadurch gekennzeichnet, daß** das m-Phenylendiaminderivat ausgewählt ist aus 2,4-Diamino-1-(2'-hydroxyethoxy)benzol, 2,4-Diamino-1-(2',3'dihydroxypropoxy)- benzol, 1,3-Bis(2',4'-diaminophenoxy)propan, 2-Amino-4-[(2'-hydroxyethyl)amino]anisol, 1,5-Bis(2'hydroxyethoxy)-2,4-diaminobenzol und m-Phenylendiamin.

5. Haarfarbträgermasse gemäß einem der Ansprüche 1 bis 4, **dadurch gekennzeichnet, daß** zusätzlich weitere Oxidationsfarbvorstufen wie p-Phenylendiaminderivate, Resorcinderivate, 1,3-Benzodioxolderivate und Naphthalinderivate enthalten sind.

6. Haarfarbträgermasse gemäß einem der Ansprüche 1 bis 5, **dadurch gekennzeichnet, daß** zusätzlich direktziehende Farbstoffe enthalten sind.

7. Haarfarbträgermasse gemäß einem der Ansprüche 1 bis 6, **dadurch gekennzeichnet, daß** die Haarfarbstoffe in einem geeigneten kosmetischen Träger angewendet werden.

8. Haarfarbträgermasse gemäß einem der Ansprüche 1 bis 7, **dadurch gekennzeichnet, daß** die Gesamtkonzentration der Farbstoffe 0,1 bis10 Gewichtsprozent beträgt.

9. Haarfarbträgermasse gemäß einem der Ansprüche 1 bis 8, **dadurch gekennzeichnet, daß** darin weitere übliche kosmetische Zusätze enthalten sind.

10. Haarfärbemittel, **dadurch gekennzeichnet, daß** es unmittelbar vor der Anwendung durch Vermischen einer Haarfarbträgermasse gemäß einem der Ansprüche 1 bis 9 mit einem Oxidationsmittel erhalten wird.

11. Haarfärbemittel nach Anspruch 10, **dadurch gekennzeichnet, daß** die Haarfarbträgermasse mit dem Oxidationsmittel in einem Verhältnis von 5:1 bis 1: 3 vermischt wird.

12. Haarfärbemittel nach Anspruch 10 oder 11, **dadurch gekennzeichnet, daß** der pH-Wert des Haarfärbemittels gleich 3 bis 11 ist.

13. Verfahren zum Färben von Haaren, **dadurch gekennzeichnet, daß** ein Haarfärbemittel nach einem der Ansprüche 10 bis 12 auf das Haar aufgetragen wird, bei einer Temperatur von 15 bis 50°C 10 bis 45 Minuten lang einwirken gelassen wird, das Haar anschließend mit Wasser gespült wird, gegebenenfalls schamponiert wird und sodann getrocknet wird.

## Claims

1. Hair colour carrier mass comprising

- at least one 4,5-diaminopyrazole according to the general formula (I),

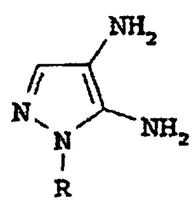

I

,

in which R is a straight-chain or branched alkyl group having 1 to 6 carbon atoms, a straight-chain or branched mono- or polyhydroxyalkyl group having 2 to 6 carbon atoms or a straight-chain or branched mono- or polyaminoalkyl group, or salts thereof with cosmetically compatible acids;

- at least one 5-amino-2-methylphenol derivative according to the general formula (II),

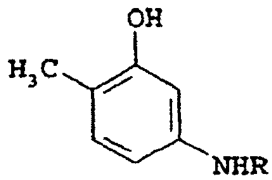

II

,

in which the radical R' has the meaning of hydrogen, a straight-chain or branched alkyl group having 1 to 4 carbon atoms, a straight-chain or branched hydroxyalkyl group having 2 to 4 carbon atoms or a straight-chain or branched polyhydroxyalkyl group having 3 to 4 carbon atoms;

- at least one m-phenylenediamine derivative according to the general formula (III),

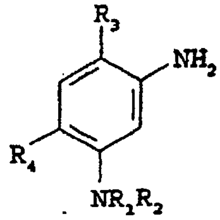

III

,

in which $R_1$ and $R_2$, independently of one another, are a hydrogen atom, a straight-chain or branched alkyl group having 1 to 4 carbon atoms, a straight-chain or branched hydroxyalkyl group having 2 to 4 carbon atoms or a straight-chain or branched polyhydroxyalkyl group having 3 to 4 carbon atoms,
$R_3$ is a hydrogen atom, a straight-chain or branched alkyl group having 1 to 6 carbon atoms, a straight-chain or branched alkoxy group having 1 to 4 carbon atoms, a straight-chain or branched hydroxyalkyl group having 2 to 4 carbon atoms or a straight-chain or branched polyhydroxyalkyl group having 3 to 4 carbon atoms, and
$R_4$, independently of $R_1$, $R_2$ and $R_3$, is a hydrogen atom, a straight-chain or branched alkyl group having 1 to 6 carbon atoms, a straight-chain or branched alkoxy group having 1 to 4 carbon atoms, a straight-chain or branched hydroxyalkyl group having 2 to 4 carbon atoms or a straight-chain or branched polyhydroxyalkyl group having 3 to 4 carbon atoms.

2. Hair colour carrier mass according to Claim 1, **characterized in that** the 4,5-diaminopyrazole derivative is chosen from 4,5-diamino-1-methyl-1H-pyrazole, 4,5-diamino-1-ethyl-1H-pyrazole, 4,5-diamino-1-(1'-methylethyl)-1H-pyrazole and 4,5-diamino-1-(2'-hydroxyethyl)-1H-pyrazole.

3. Hair colour carrier mass according to Claim 1, **characterized in that** the 5-amino-2-methylphenol derivative is chosen from 5-amino-2-methylphenol, 5-methylamino-2-methylphenol and 5-[(2'-hydroxyethyl)amino]-2-methylphenol.

4. Hair colour carrier mass according to Claim 1, **characterized in that** the m-phenylenediamine derivative is chosen from 2,4-diamino-1-(2'-hydroxyethoxy)benzene, 2,4-diamino-1-(2',3'-dihydroxypropoxy)benzene, 1,3-bis(2',4'-diaminophenoxy)propane, 2-amino-4-[(2'-hydroxyethyl)amino]anisole, 1,5-bis(2'-hydroxyethoxy)-2,4-diaminobenzene and m-phenylenediamine.

5. Hair colour carrier mass according to one of Claims 1 to 4, **characterized in that** further oxidation dye precursors, such as p-phenylenediamine derivatives, resorcinol derivatives, 1,3-benzodioxole derivatives and naphthalene derivatives, are additionally present.

6. Hair colour carrier mass according to one of Claims 1 to 5, **characterized in that** direct dyes are additionally present.

7. Hair colour carrier mass according to one of Claims 1 to 6, **characterized in that** the hair dyes are applied in a suitable cosmetic carrier.

8. Hair colour carrier mass according to one of Claims 1 to 7, **characterized in that** the total concentration of the dyes is 0.1 to 10 per cent by weight.

9. Hair colour carrier mass according to one of Claims 1 to 8, **characterized in that** further customary cosmetic additives are present therein.

10. Hair colorant **characterized in that** it is obtained directly prior to application by mixing a hair colour carrier mass according to one of Claims 1 to 9 with an oxidizing agent.

11. Hair colorant according to Claim 10, **characterized in that** the hair colour carrier mass is mixed with the oxidizing agent in a ratio of 5:1 to 1:3.

12. Hair colorant according to Claim 10 or 11, **characterized in that** the pH of the hair colorant is 3 to 11.

13. Method of colouring hair, **characterized in that** a hair colorant according to one of Claims 10 to 12 is applied to the hair, left to act at a temperature of from 15 to 50°C for 10 to 45 minutes, the hair is then rinsed with water, optionally shampooed and then dried.

**Revendications**

1. Matière colorante pour cheveux, contenant

- au moins un 4,5-diaminopyrazole de formule générale (I)

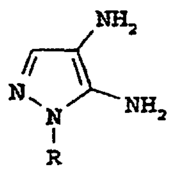

I

dans laquelle R représente un groupe alkyle à chaîne droite ou ramifiée ayant de 1 à 6 atomes de carbone, un groupe mono- ou polyhydroxyalkyle à chaîne droite ou ramifiée ayant de 2 à 6 atomes de carbone ou un groupe mono- ou polyaminoalkyle à chaîne droite ou ramifiée, ou ses sels avec des acides cosmétiquement acceptables ;

- au moins un dérivé de 5-amino-2-méthylphénol de formule générale (II)

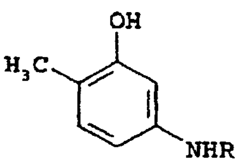

II

dans laquelle le radical R' représente un atome d'hydrogène, un groupe alkyle à chaîne droite ou ramifiée ayant de 1 à 4 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone,

- au moins un dérivé de m-phénylènediamine de formule générale (III)

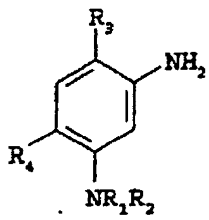

III

dans laquelle $R_1$ et $R_2$ représentent, indépendamment l'un de l'autre, un atome d'hydrogène, un groupe alkyle à chaîne droite ou ramifiée ayant de 1 à 4 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone,
$R_3$ représente un atome d'hydrogène, un groupe alkyle à chaîne droite ou ramifiée ayant de 1 à 6 atomes de carbone, un groupe alcoxy à chaîne droite ou ramifiée ayant de 1 à 4 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone, et
$R_4$ représente, indépendamment de $R_1$, $R_2$ et $R_3$, un atome d'hydrogène, un groupe alkyle à chaîne droite ou ramifiée ayant de 1 à 6 atomes de carbone, un groupe alcoxy à chaîne droite ou ramifiée ayant de 1 à 4 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone.

2. Matière colorante pour cheveux selon la revendication 1, **caractérisée en ce que** le dérivé de 4,5-diaminopyrazole est choisi parmi le 4,5-diamino-1-méthyl-1H-pyrazole, le 4,5-diamino-1-éthyl-1H-pyrazole, le 4,5-diamino-1-(1'-méthyléthyl)-1H-pyrazole et le 4,5-diamino-1-(2'-hydroxyéthyl)-1H-pyrazole.

3. Matière colorante pour cheveux selon la revendication 1, **caractérisée en ce que** le dérivé de 5-amino-2-méthylphénol est choisi parmi le 5-amino-2-méthylphénol, le 5-méthylamino-2-méthylphénol et le 5-[(2'-hydroxyéthyl)amino]-2-méthylphénol.

4. Matière colorante pour cheveux selon la revendication 1, **caractérisée en ce que** le dérivé de m-phénylènediamine est choisi parmi le 2,4-diamino-1-(2'-hydroxyéthoxy)benzène, le 2,4-diamino-1-(2',3'-dihydroxypropoxy)benzène, le 1,3-bis(2',4'-diaminophénoxy)propane, le 2-amino-4-[(2'-hydroxyéthyl)amino]anisole, le 1,5-bis(2'-hydroxyéthoxy)-2,4-diaminobenzène et la m-phénylènediamine.

5. Matière colorante pour cheveux selon l'une quelconque des revendications 1 à 4, **caractérisée en ce qu'**elle contient en outre d'autres précurseurs de colorants d'oxydation, tels que des dérivés de p-phénylènediamine, des dérivés de résorcinol, des dérivés de 1,3-benzodioxole et des dérivés de naphtalène.

6. Matière colorante pour cheveux selon l'une quelconque des revendications 1 à 5, **caractérisée en ce qu'**elle contient en outre des colorants directs.

7. Matière colorante pour cheveux selon l'une quelconque des revendications 1 à 6, **caractérisée en ce que** les colorants pour cheveux sont utilisés dans un véhicule cosmétique approprié.

8. Matière colorante pour cheveux selon l'une quelconque des revendications 1 à 7, **caractérisée en ce que** la concentration totale des colorants va de 0,1 à 10 % en poids.

9. Matière colorante pour cheveux selon l'une quelconque des revendications 1 à 8, **caractérisée en ce qu'**elle contient d'autres additifs cosmétiques usuels.

10. Produit de teinture pour cheveux, **caractérisé en ce qu'**il est préparé immédiatement avant l'emploi, par mélange d'une matière colorante pour cheveux selon l'une quelconque des revendications 1 à 9, avec un oxydant.

11. Produit de teinture pour cheveux selon la revendication 10, **caractérisé en ce que** la matière colorante pour cheveux est mélangée avec l'oxydant en un rapport de 5:1 à 1:3.

12. Produit de teinture pour cheveux selon la revendication 10 ou 11, **caractérisé en ce que** le pH du produit de

teinture pour cheveux vaut de 3 à 11.

**13.** Procédé de teinture des cheveux, **caractérisé en ce qu'**on applique sur les cheveux un produit de teinture pour cheveux selon l'une quelconque des revendications 10 à 12, on laisse agir pendant 10 à 45 minutes à une température de 15 à 50°C, puis on rince les cheveux à l'eau, éventuellement on les shampooine et ensuite on les sèche.